# EUROPEAN PATENT APPLICATION

(11) **EP 2 962 561 A1**
(43) Date of publication of application: **06.01.2016**
(21) Application number: 14757456.0
(22) Date of filing: 28.02.2014
(51) Int. Cl.: A01N 25/02, A01N 25/12, A01N 59/16, A01P 3/00

(54) **PROCESS FOR PRODUCING SILVER-ION ANTIBACTERIAL LIQUID, SILVER-ION ANTIBACTERIAL LIQUID PRODUCED BY SAID PROCESS, AND SILVER-ION-CONTAINING PRODUCT CONTAINING SAID ANTIBACTERIAL LIQUID**

(30) Priority: 28.02.2013 JP 2013039486; 27.02.2014 JP 2014037138
(71) Applicant: Taiki Corp., Ltd., Osaka-shi, Osaka 534-0014 (JP)
(72) Inventor: NAKAMURA Kenji, Osaka-shi Osaka 533-0031 (JP); NAKAMURA Koji, Osaka-shi Osaka 533-0031 (JP)
(74) Representative: Tischner, Oliver
(86) International application number: PCT/JP2014/055116
(87) International publication number: WO 2014/133149

(57) **Abstract**

The present invention aims to provide a method for producing a silver ion antibacterial liquid that does not include a silica hydrate removal process but still eliminates the risk of discoloration and is also trouble-free, as well as a silver ion antibacterial liquid produced by such method and a silver ion-containing product that contains such antibacterial liquid. As the means for solving the problems a method for producing silver ion antibacterial liquid is proposed where the silver ion antibacterial liquid to be produced contains silver ions eluted from a silver zeolite, wherein said method for producing silver ion antibacterial liquid is characterized in that it involves: using the silver zeolite, citric acid, and purified water, and implementing a first process comprising weighing the silver zeolite, citric acid, and purified water and taking a blending quantity of each, and then agitating and mixing a blended liquid prepared by blending the silver zeolite, citric acid, and purified water to prepare a mixture liquid, or a second process comprising weighing and taking a blending quantity of each of the above, preparing the mixture liquid mentioned above, and then adding purified water to dilute the mixture liquid and prepare a diluted liquid; wherein, the blending ratio of the citric acid to the silver zeolite is 0.9 to 1.5 in the first or second process.

## Description

### Technical Field

The present invention relates to a method for producing silver ion antibacterial liquid that involves using a silver zeolite as highly safe, low-cost material, collapsing its crystal structure using citric acid, and eluting silver ions contained in its crystal structure into a liquid, thereby allowing for the resulting silver ion antibacterial liquid to be produced in large quantities, as well as a silver ion antibacterial liquid produced by such method, and a silver ion-containing product that contains such antibacterial liquid.

### Background Art

Bacteria are said to produce odor by breaking down human secretions. For example, the cause of underarm odor is the sweat secreted from the apocrine sweat glands in the skin; specifically when this sweat is secreted onto the skin, it is mixed with the fat secreted from the sebaceous glands and sweat secreted from the eccrine sweat glands, and as the resulting mixture is broken down by the indigenous bacteria on the skin or underarm hair, a substance that releases underarm odor is produced. The above indigenous bacteria on the skin include Staphylococcus aureus and Propionibacterium acnes, while the odorous components include butyric acid and valeric acid. Types of odor include underarm odor, sweat odor and hair odor.

General types of odor are largely classified into three types including fatty acid odor (body odor, sweat, etc.), nitrogen compound odor (decayed urine, etc.) and sulfur compound odor (excrement, etc.). Means for preventing these odors include (1) masking using fragrance, (2) adsorption using active carbons, zeolites, etc., (3) neutralization using acids and alkalis, and (4) killing of bacteria using antibacterial agents. The masking in (1) does not offer a fundamental solution which is effective in preventing the odor because, although the odor is temporarily suppressed as the fragrance volatilizes, the foul smell will come back. Adsorption in (2) presents a problem in terms of odor elimination effectiveness because the adsorption capability is limited. Neutralization in (3) presents a problem in that it can be applied only for specific odors. As for killing of bacteria using antibacterial agents in (4), while some antibacterial agents trigger allergic reaction or cause irritation and are not desirable, silver-based inorganic antibacterial agents (silver zeolites) are recognized for their high safety, antibacterial spectrum, sustained effectiveness, etc., and are used in antibacterial liquids, deodorizing liquids, cosmetics, sanitary products, etc., for example.

Also, various inventions relating to silver zeolites have been proposed.

For example, a deodorizing cosmetic material is proposed that contains silicone and an antibacterial zeolite whose ion-exchangeable ions have been partially or completely ion-exchanged with metal ions such as zinc ions, ammonium ions, and silver ions, and the antibacterial zeolite constituting this deodorizing cosmetic material (Zeomic AJ10N manufactured by Sinanen Zeomic Co., Ltd., whose average particle size is 2.5 µm) (weight of silver ions supported on silver zeolite: 2.2 percent by weight) is suggested for use in an aerosol form (refer to Patent Literature 1). There have also been attempts to suppress discoloration due to the silver ions, one of which is a proposal of an antibacterial zeolite offering excellent resistance to discoloration, prepared by blending ammonium ions and a silver zeolite whose zeolite has been substituted by silver ions (refer to Patent Literature 2).

Patent Literature 3 is also known that points out that the silver zeolites mentioned above do not have immediate effect. Patent Literature 3 describes a zeolite-based antibacterial agent containing heavy metal ions such as zinc, silver, and copper ions as an antibacterial agent whose antibacterial effect against bacteria and fungi lasts for a long period of time. As for the types of heavy metal ions, silver ions are widely used in recent years due to their superiority, particularly in the area of safety. As for the bactericidal power efficacy and deodorizing power efficacy immediately after the treatment, silver ions do not provide sufficient bactericidal performance compared to chlorine-based bactericides and other oxidizing agents, and to solve this problem, in place of a zeolite-based antibacterial agent, an antibacterial agent is proposed that contains a silver-chloro complex salt and oxidizing agent (refer to Patent Literature 3). However, it is clear that, so long as fast-acting silver ions can be produced from a silver zeolite, then odor-generating bacteria can be killed and deodorization can be achieved as a result.

These silver zeolites mentioned above represent inventions that utilize the elution of silver ions by silver zeolite, and have a three-dimensional framework structure based on alumino-silicate-namely, a three-dimensional framework structure of Si-O-Al-O-Si where silicon (Si) and aluminum (Al) are bonded via oxygen (O)-and because aluminum (valence of +3) and silicon (valence of +4) mutually share oxygen (valence -2), the vicinity of silicon is electrically neutral, while the vicinity of aluminum has a valence of -1 (Al⁻). To compensate for this negative electric charge, normally sodium ions (Na⁺) are retained. The aforementioned silver zeolites have some of the sodium ions in the aforementioned framework substituted with silver ions (Ag⁺) having antibacterial property. Their structure is such that these silver ions are electrostatically bonded in the framework and this very structure reportedly explains the excellent sustained release performance (performance of demonstrating antibacterial action over an extended period of time) of these silver zeolites as it causes the silver ions to elute as a result of ion exchange action and consequently kill bacteria.

However, the aforementioned silver zeolites need a long time to produce their effects because utilizing the elution of silver ions resulting from ion exchange with cations in the water means that bacteria are killed by silver ions that elute only gradually. In other words, it has been pointed out that they do not have immediate effect to kill bacteria quickly (refer to Non-patent Literature 1).

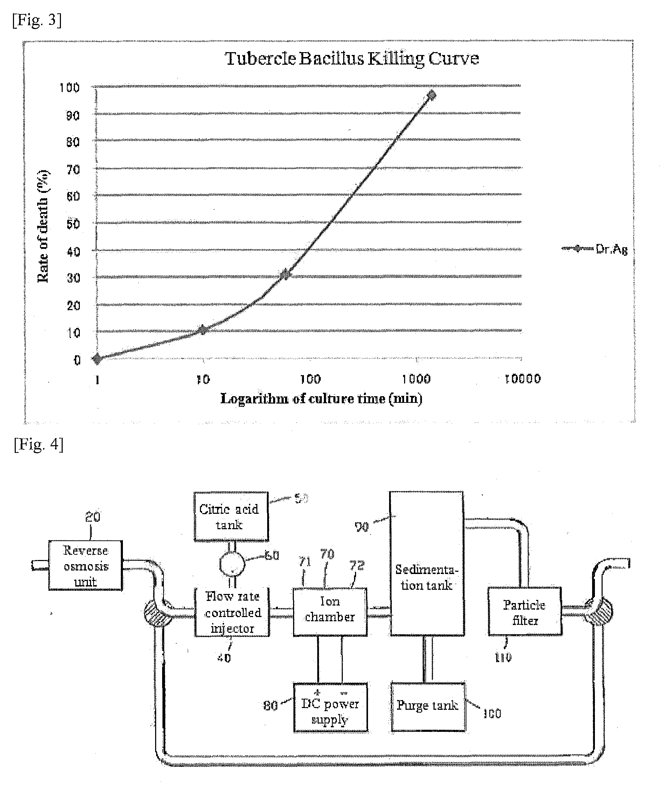

Patent Literature 4 proposes a water-based bactericide having immediate bactericidal effect, produced by using an electrolysis system. It is described that this water-based bactericide can be produced as a citric acid-silver complex from silver ions generated in an aqueous solution of citric acid using an electrolysis system with silver electrodes, and from the citric acid. As shown in Fig. 4, the electrolysis system comprises a flow-rate controlled injector 40, citric acid tank 50, ion chamber 70, DC power supply 80, sedimentation tank 90, purge tank 100 and particle filter 110. An anode 71 and cathode 72 are installed in the ion chamber 70, where the anode 71 and cathode 72 are placed away from each other so that a diluted citric acid solution can pass between the anode 71 and cathode 72. The anode 71 and cathode 72 are each formed from silver of 99.9999% purity. Additionally, when a sample was measured by the nuclear magnetic resonance test (1H-NMR) to examine the chemical structure of silver ions generated by the electrolysis system, the sample was overwhelming rich in citric acid and other anions were hardly present, which indicates compounding of complex bonds with respect to silver ions as mentioned (refer to Patent Literature 4). This suggests that it is difficult to identify the specific structure of a complex produced from silver ions and citric acid.

As mentioned above, Patent Literature 4 discloses that a citric acid-silver complex can be produced in an aqueous solution of citric acid using an electrolysis system having silver electrodes.

Additionally, Ciba Specialty Chemicals sells a solution containing the aforementioned citric acid-silver complex under the brand name TINOSAN SDC (brand name), and the INCI (International Nomenclature of Cosmetic Ingredients) designation of this solution is Citric Acid and Silver Citrate. It is reported that TINOSAN SDC (brand name) is an antibacterial silver for skincare use, which is a silver complex produced from silver and citric acid through a unique electrical process (refer to Non-patent Literature 2).

The citric acid-silver complex described in Patent Literature 4 is produced by an electrolysis system comprising a flow-rate controlled injector 40, citric acid tank 50, ion chamber 70, DC power supply 80, sedimentation tank 90, purge tank 100 and particle filter 110, in a container that fills a diluted citric acid solution between the anode and cathode formed by high-purity silver in the ion chamber 70. Accordingly, among other costs the high equipment cost of installing the electrolysis system, and the high maintenance cost associated with the replacement of the anode and cathode formed by high-purity silver due to wear, make it an expensive way to produce the citric acid-silver complex and lowering these costs is difficult. In the case of TINOSAN SDC (brand name), which is a silver complex reportedly produced by a unique electrical process, its production method used is probably similar to the method for producing a citric acid-silver complex using an electrolysis system and silver electrodes as described in Patent Literature 4.

Also, TINOSAN SDC (brand name), which is sold on the market, is recognized as an excellent antibacterial agent for skincare use because it does not contain paraben as a preservative nor alcohol, so it can be used by anyone with peace of mind as a paraben-free, alcohol-free antibacterial agent for skincare use. However, this product is not popular because it is too expensive for everyday use by general consumers. Accordingly, there is a need for a low-cost method to produce a silver ion antibacterial liquid containing a citric acid-silver complex.

In light of the problems of the prior arts mentioned above, the inventors of the present invention attempted, through continuous research in earnest, to solve the problems and completed the invention entitled, "Method for Producing Silver Ion Antibacterial Liquid" that allows for production of a citric acid-silver complex by mixing silver zeolite and citric acid at a specific blending ratio, thereby collapsing the crystal structure of the entire silver zeolite. The invention is a method for producing silver ion antibacterial liquid comprising: a process to weigh and take a blending quantity of silver zeolite and then weigh citric acid and take a blending quantity of it so that its blending ratio to the silver zeolite becomes 1.2 or greater, followed by blending of the two into purified water; a process to mix under agitation the silver zeolite and citric acid blended in the purified water to prepare a mixture liquid containing at least a citric acid-silver complex and silica hydrate; and a process to remove the silica hydrate produced in the mixture liquid, and its application was filed (on September 1, 2011) with the Japanese Patent Office as Toku-Gan 2011-191039 (Japanese Patent Laid-open No. 2013-053085). According to the method for production under the invention of the prior application for patent, the products generated by the aforementioned mixture liquid preparation process include not only citric acid-silver complex, but also silica hydrate, but since the silica hydrate may become silica hydrate silver hydroxide due to silver hydroxide adsorbed onto its surface, and agglutinate as a result, and this agglutinated product may turn into silver oxide (of blackish brown color) if light is irradiated onto it, the process to remove the silica hydrate from the mixture liquid is included.

### Prior Art Literatures

### Patent Literatures

Patent Literature 1: Japanese Patent Laid-open No. Hei 08-092051
Patent Literature 2: Japanese Patent Laid-open No. Sho 63-265809
Patent Literature 3: PCT International Patent Laid-open No. 99/065317
Patent Literature 4: Published Japanese Translation of PCT International Patent Application No. 2001-519361

### Non-patent Literatures

Non-patent Literature 1: "Jintai Jozai Kin no Hanashi (A Story of Indigenous Bacteria in Human Body)," Noboru Aoki, Shueisha Shinsho, pp. 182-183, October 29, 2008 (seventh reprint)
Non-patent Literature 2: http://naturalingredient.org/Articles/Tinosan_Micro_info.pdf

### Summary of the Invention

### Problems to Be Solved by the Invention

The silver ion antibacterial liquid from which the silica hydrate has been removed according to the method for producing silver ion antibacterial liquid mentioned above, does not change color even if stored for a long period of time and also allows for selection of a desired silver ion concentration freely according to any of its various applications for use. Accordingly, the inventors of the present invention used a pilot plant to gather data of yield, production output, etc., in order to mass-produce the silver ion antibacterial liquid using the method for production mentioned above. The silica hydrate can be removed by: (1) decanting the deposited agglutinated silica hydrate silver hydroxide; (2) filtering the deposited agglutinated silica hydrate silver hydroxide ; (3) filtering the silica hydrate before it agglutinates; or (4) adding a divalent metal salt (such as zinc citrate) when the silver zeolite and citric acid are blended in the purified water, thereby causing the divalent metal ions to bond with the silica hydrate and deposit together, for easy removal of the silica hydrate. For example, when the filtering in (3) above was implemented using the Watman CF/C filter paper, the filter paper clogged up quickly and had to be changed, revealing that removing the silica hydrate this way would take time and trouble.

In light of the problems of the invention under the prior application for patent mentioned above, the present invention aims to provide a method for producing silver ion antibacterial liquid that does not include a silica hydrate removal process but still eliminates the risk of discoloration and is also trouble-free, as well as a silver ion antibacterial liquid produced by such method and a silver ion-containing product that contains such antibacterial liquid.

### Means for Solving the Problems

After studying repeatedly in earnest for a method for producing silver ion antibacterial liquid that does not cause discoloration and is trouble-free, the inventors completed the present invention by finding that, by using a silver zeolite, citric acid, and purified water, and implementing a first process comprising weighing the silver zeolite, citric acid, and purified water, and taking a blending quantity of each, and then agitating and mixing into the purified water a blended liquid prepared by blending the silver zeolite and citric acid to prepare a mixture liquid, or a second process comprising weighing and taking a blending quantity of each of the above, preparing the mixture liquid mentioned above, and then adding purified water to dilute the mixture liquid and prepare a diluted liquid, and, if the blending ratio of the citric acid to the silver zeolite corresponds to a blending quantity in a range of 0.9 to 1.5 after the first process or second process, and if the silver ion antibacterial liquid contains fast-acting silver ions and the concentration of the silver ions is adjusted to an appropriate range, then the antibacterial liquid does not turn blackish brown due to light.

In other words, the present invention is described below:
1. A method for producing silver ion antibacterial liquid where the silver ion antibacterial liquid to be produced contains silver ions eluted from a silver zeolite supporting 0.5 to 5.0 percent by weight of silver, wherein said method for producing silver ion antibacterial liquid is characterized in that it involves:
   using the silver zeolite, citric acid, and purified water, and implementing a first process comprising weighing the silver zeolite, citric acid, and purified water, and taking a blending quantity of each, and then agitating and mixing a blended liquid prepared by blending the silver zeolite, citric acid, and purified water to prepare a mixture liquid, or a second process comprising weighing and taking a blending quantity of each of the above, preparing the mixture liquid mentioned above, and then adding purified water to dilute the mixture liquid and prepare a diluted liquid;
   wherein, the blending ratio of the citric acid to the silver zeolite corresponds to a blending quantity in a range of 0.9 to 1.5 when the mixture liquid is prepared in the first and second process, and the silver ion concentration of the produced silver ion antibacterial liquid is 2.5 to 750 ppm.
2. A method for producing silver ion antibacterial liquid according to 1, characterized in that the blending quantity of the silver zeolite is 0.01 to 3.0 percent by weight in the first process, while the blending quantity of the silver zeolite exceeds 3.0 percent by weight in the second process.
3. A method for producing silver ion antibacterial liquid according to 1, characterized in that the silver zeolite is a type A or type X silver zeolite.
4. A silver ion antibacterial liquid produced by a method for producing silver ion antibacterial liquid according to 1.
5. A silver ion antibacterial powder characterized in that a silver ion antibacterial liquid according to 1 is freeze-dried or spray-dried and powderized.
6. A silver ion-containing product characterized in that a silver ion antibacterial liquid according to 4 is blended into a product selected from the group that includes liquid makeup preparations, wet tissues, liquid deodorants, liquid bath agents and liquid laundry softeners.
7. A silver ion-containing treated product characterized in that a silver ion antibacterial liquid according to 4 is coated or deposited onto an article used in hospitals and public places.

### Effects of the Invention

The method for producing silver ion antibacterial liquid as proposed by the present invention does not use the electrolysis system and silver electrodes mentioned above, but uses instead a low-cost type A or type X silver zeolite on which silver ions are supported, and citric acid, as materials to produce a silver ion antibacterial liquid containing a citric acid-silver complex, which substantially lowers the manufacturing cost, and the silver ion antibacterial liquid demonstrates an immediate bactericidal effect to kill bacteria quickly.

In addition, the method for producing silver ion antibacterial liquid as proposed by the present invention is such that, so long as the blending quantity of silver zeolite is determined, the blending quantity of citric acid can be determined easily as a quantity corresponding to a blending ratio in a range of 0.9 to 1.5, and also because the process for producing silver ion antibacterial liquid comprises a process to weigh and take a blending quantity of each material and a process to prepare a mixture liquid or process to complete the foregoing processes and then prepare a diluted liquid, the silver ion antibacterial liquid can be prepared with simple processing operations and no process is needed to remove the silica hydrate produced in the mixture liquid unlike under the invention of the prior application for patent. In addition, the antibacterial liquid does not change color to blackish brown when irradiated with light.

As for the method for producing silver ion antibacterial liquid as proposed by the present invention, the first process is suitable for small-volume production of silver ion antibacterial liquid, while the second process is suitable for mass-production thereof.

The silver ion antibacterial liquid as proposed by the present invention allows for its silver ion concentration to be adjusted in a desired manner according to its application for use based on the blending quantity of, and quantity of silver supported on, silver zeolite, as well as the blending quantity of purified water, and also because it is inexpensive, the silver ion antibacterial liquid can be used by general consumers as a popular product and also, for example, as a paraben-free, alcohol-free antibacterial agent for skincare purpose anyone can use with peace of mind.

In addition, the silver ion antibacterial liquid as proposed by the present invention has an immediate bactericidal effect to kill bacteria quickly, which was not possible with the ion exchange action of any conventional silver-supporting zeolite.

The silver ion-containing product as proposed by the present invention does not change color to darkish brown and permits its quality to be guaranteed for a long period of time.

### Brief Description of the Drawings

[Fig. 1] is a graph showing the bactericidal killing rate against each bacterium achieved by a silver ion antibacterial liquid (silver ion concentration: 10 ppm) according to the present invention.
[Fig. 2] is a graph showing how the bacterial count changes over time when a silver ion antibacterial liquid (silver ion concentration: 10 ppm) according to the present invention, and a control sterile saline solution, are caused to act upon the Tubercle bacillus.
[Fig. 3] is a graph showing a killing curve of the Tubercle bacillus when a silver ion antibacterial liquid (silver ion concentration: 10 ppm) according to the present invention is caused to act upon it.
[Fig. 4] is a diagram showing the structure of a conventional electrolysis system for producing a citric acid-silver complex.

### Mode for Carrying Out the Invention

### (Most Preferred Embodiment)

The silver zeolite used under the present invention is either a type A or type X zeolite (any such silver zeolite is hereinafter simply referred to as "silver zeolite"). Since type X silver zeolites are expensive, preferably a type A silver zeolite is used. This type A and type X silver zeolite are dissolved by an acid, which is why the present invention uses these two zeolites. On the other hand, type Y silver zeolites and mordenite silver zeolites do not dissolve in acids and thus cannot be used. The structural formula of silver zeolite is shown below.

(αNa₂ βAg₂)O · Al₂O₃-2SiO₂nH₂O (α + β = 1, n = 5; dried at 110°C)

The crystal structure forming the ion exchange site of the above silver zeolite is a three-dimensionally bonded Si-O-Al-O-Si crystal structure whose Al has the silver ion electrostatically bonded to it, and reportedly the silver ion in the above crystal structure elutes as a result of ion exchange action to kill bacteria.

### (Method for Manufacturing Silver Zeolite)

The method for manufacturing silver zeolite is explained below.

The explanation is based on type A zeolite as an example of the material, but the same manufacturing procedure for type A zeolite applies when a type X zeolite is manufactured. It should be noted that the method for manufacturing silver zeolite explained below is a traditionally known manufacturing method.

Put water in a plastic container and introduce a type A zeolite (Na type) therein little by little and agitate to prepare a suspension liquid, and then release air from the solids. Check the pH after an elapse of the specified time. Add diluted nitric acid (diluted by 6 times) by a small quantity at a time to adjust the pH to a range of 5 to 7, and use a pH litmus paper to check the pH change roughly.

Separately, pre-mix silver nitrate with water and introduce the mixture into the type A zeolite slurry little by little under agitation. Thereafter, let it stand under agitation overnight. Install a magnetic funnel on a Nutsche, put in a standard filter paper, and slowly pour the silver zeolite slurry onto the filter paper. Before the liquid runs out in the suction process, wash the filtrate with water.

The method for manufacturing three types of silver zeolite supporting 0.5 percent by weight of silver (Manufacturing Example 1), 2.5 percent by weight of silver (Manufacturing Example 2) and 5.0 percent by weight of silver (Manufacturing Example 3), respectively, is explained below as manufacturing examples.

Manufacturing Example 1. Silver zeolite supporting 0.5 percent by weight of silver
(1) Material
   Type A zeolite (dried at 110°C): 1000 g
   Silver nitrate (AgNO₃): 7.9 g
(2) Manufacturing procedure
   Put 4.0 L of water in a 10-L plastic container and introduce a type A zeolite (Na type) therein little by little and agitate to produce a suspension liquid. Agitate the liquid continuously for 3 hours or so, to release air from the solids.
   Check the pH after an elapse of the specified time. Add diluted nitric acid (diluted by 6 times) by a small quantity at a time to adjust the pH to a range of 5 to 7, and use a pH litmus paper to check the pH change roughly.
   Pre-mix silver nitrate with 3.0 L of water separately and introduce the mixture into the type A silver zeolite slurry little by little under agitation. Let it stand under agitation overnight.
   Install a magnetic funnel on a Nutsche, put in a standard filter paper, and slowly pour the silver zeolite slurry onto the filter paper.
   Before the solution runs out in the suction process, wash the filtrate with 5 L of water.
   Let the filtrate dry overnight at 110°C and then cool and crush the dried filtrate into powder in a mortar. This gives a type A silver zeolite in powder state, having an average particle size of 2 to 2.5 µm.

Manufacturing Example 2. Silver zeolite supporting 2.5 percent by weight of silver
(1) Material
   Type A zeolite (dried at 110°C): 1000 g
   Silver nitrate (AgNO₃): 39.7 g
(2) The manufacturing procedure is the same as the one described above.

Manufacturing Example 3. Silver zeolite supporting 5.0 percent by weight of silver
(1) Material
   Type A zeolite (dried at 110°C): 1000 g
   Silver nitrate (AgNO₃): 79.5 g
(2) The manufacturing procedure is the same as the one described above.

### (Method for Producing Silver Ion Antibacterial Liquid)

The method for producing silver ion antibacterial liquid as proposed by the present invention involves: using the silver zeolite, citric acid, and purified water, and implementing a first process comprising weighing the silver zeolite, citric acid, and purified water, and taking a blending quantity of each, and then agitating and mixing a blended liquid prepared by blending the silver zeolite, citric acid, and purified water to prepare a mixture liquid, or a second process comprising weighing and taking a blending quantity of each of the above, preparing the mixture liquid mentioned above, and then adding purified water to dilute the mixture liquid and prepare a diluted liquid; wherein, the blending ratio of the citric acid to the silver zeolite corresponds to a blending quantity in a range of 0.9 to 1.5 in the first or second process, and an immediate bactericidal effect is achieved because the silver ion antibacterial liquid contains silver ions. It is appropriate that the silver ion antibacterial liquid has a silver ion concentration of 2.5 to 750 ppm to prevent discoloration to blackish brown.

The blending ratio mentioned above refers to the percentage of the blending quantity of citric acid (in percent by weight) to the blending quantity of silver zeolite (in percent by weight), or specifically the ratio of "Percent by weight of citric acid / Percent by weight of silver zeolite," and this ratio is defined as the "blending ratio" and used accordingly.

The method for producing silver ion antibacterial liquid is explained using a specific example.

### (Mixture Liquid Blending Process)

Determine the blending quantities of silver zeolite, citric acid and purified water beforehand, respectively, based on the desired quantity of silver ion antibacterial liquid to be produced.

The blending quantity of silver zeolite is 0.01 to 3.0 percent by weight of the desired quantity of silver ion antibacterial liquid to be produced.

Also weigh beforehand the citric acid and take the blending quantity of it so that the citric acid accounts for 0.9 to 1.5 times the weight of the silver zeolite.

Blend the silver zeolite and citric acid into the purified water of the aforementioned blending quantity at normal temperature (28°C) to prepare a blended liquid. This blended liquid in which the two materials are blended together appears cloudy immediately after the blending. Thereafter, agitate the liquid until the cloudy liquid turns translucent or clear, to produce a mixture liquid. If the blending ratio of the citric acid corresponds to a blending quantity of 0.9 or greater but less than 1.2, a translucent mixture liquid should be obtained after at least 10 minutes of mixing under agitation. If the blending quantity of the citric acid corresponds to a blending quantity of 1.2 or greater but no greater than 1.5, a clear mixture liquid should be obtained after at least 2 minutes of mixing under agitation. Or, the silver zeolite may be blended into the purified water at ordinary temperature to prepare a blended liquid, followed by blending the citric acid into the purified water at ordinary temperature in a similar manner to prepare a blended liquid, after which the two liquids may be mixed under agitation to produce a mixture liquid.

Here, Zeomic AJ10N (manufactured by Sinanen Zeomic Co., Ltd.) supporting 2.2 percent by weight of silver can be used as the silver zeolite.

Next, the products generated by the mixture liquid preparation process are explained.

The mixture liquid prepared by blending the silver zeolite ((αNa₂ βAg₂)O · Al₂O₃-2SiO₂nH₂O (α + β = 1, n = 5; dried at 110°C)) and citric acid (C₆H₈O₇) in the purified water under agitation contains a citric acid-silver complex, citric acid-aluminum complex, sodium ion (Na⁺), and silica hydrate in light of the chemical formulas of the two materials.

As the silver zeolite and citric acid are mixed, first the proton in the citric acid (H⁺) attacks and severs the Al-O part in the Si-O-Al-O-Si structure of the silver zeolite, and as a result the zeolite framework collapses and the ion exchange adsorption site is lost, and this causes the silver to elute into the mixture liquid.

These silver ions react with the citric acid and produce a citric acid-silver complex while a very small quantity of silver ions are produced. On the other hand, the aluminum reacts with the citric acid and produces a citric acid-aluminum complex, and besides the above, a silica hydrate and sodium ions are presumably produced.

The structural formula of the aforementioned citric acid-silver complex is as follows:

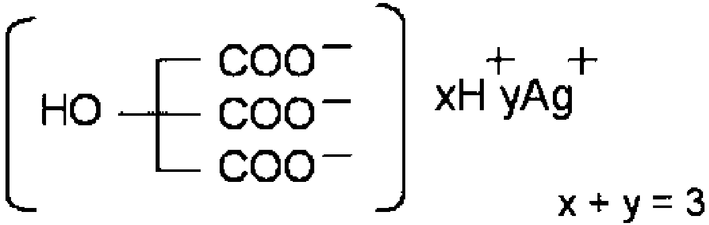

In the formula, y is 1 and/or 2, and if y is 3, the complex becomes sparingly soluble and no longer dissolves in water. The citric acid-silver complex produced by the silver ion and citric acid reacting together is a complex where y is 1 and x is 2, because a majority of the complex is citrate monosilver.

To blend silver zeolite and citric acid for the silver ion antibacterial liquid mentioned above, silver zeolite is weighed and a blending quantity of it in a range of 0.01 to 3.0 percent by weight is taken, and then citric acid is weighed and a blending quantity of it is taken so that its blending ratio to the silver zeolite becomes 0.9 to 1.5, followed by adjustment of the blending quantity using purified water. The blending quantity of silver zeolite and that of citric acid were derived from the results of the first experiment described below. The first experiment reflects that, when silver zeolite and citric acid are mixed, the proton in citric acid (H⁺) collapses the framework structure of the Al-O part in the Si-O-Al-O-Si structure of silver zeolite, thereby leading to loss of the ion exchange adsorption site and elution of silver ions into the mixture liquid. In consideration of this mechanism, the experiment was conducted by assuming that, although a liquid in which silver zeolite is dispersed is normally cloudy immediately after its mixing, the collapse of the framework structure would turn the mixture liquid clear.

Accordingly, an experiment to examine how much citric acid should be blended relative to the blending quantity of silver zeolite in order to obtain a clear mixture liquid (hereinafter referred to as the "first experiment") was conducted first.

Next, an experiment to examine whether or not all silver ions supported on the silver zeolite could be eluted (hereinafter referred to as the "second experiment") was conducted, where multiple types of silver zeolite with different supported quantities of silver were used to examine the silver ion concentrations in the mixture liquids obtained as a result of the first experiment which were produced at the aforementioned blending ratio.

### (First Experiment)

As examples of silver zeolite, a type A silver zeolite (supporting 2.5 percent by weight of silver) manufactured according to the aforementioned method for manufacturing silver zeolite, and Zeomic AJ10N (manufactured by Sinanen Zeomic Co., Ltd., supporting 2.2 percent by weight of silver), were used as samples.

Two types of the type A silver zeolite, blended by 0.5 percent by weight or 2.5 percent by weight in a silver antimicrobial liquid, respectively, were weighed and specific quantities were taken to prepare six samples each, for a total of 12 samples. Also, two types of Zeomic AJ10N, also having the aforementioned different blending quantities, respectively, were weighed and specific quantities were taken to prepare a total of 12 samples.

For the six samples each of the same blending quantity of 0.5 percent by weight or 2.5 percent by weight, citric acid was weighed and taken at a ratio of 0.8 relative to the weight of silver zeolite for Sample No. 1 and No. 7, and, similarly, of 0.9 for Sample No. 2 and No. 8, of 1.0 for Sample No. 3 and No. 9, of 1.2 for Sample No. 4 and No. 10, of 1.3 for Sample No. 5 and No. 11, and of 1.5 for Sample No. 6 and No. 12, as shown in the "Blending ratio" column in Table 1. Each of these weighed silver zeolites was blended with citric acid powder in purified water to prepare 200 g of blended liquid, and the pH of this mixture liquid was measured using a pH meter after 2 minutes, 10 minutes, and 30 minutes. The appearance of the mixture liquid was visually observed and determined on a 4-point scale of cloudy, containing deposits, translucent, and clear.

Table 1 shows the results of the first experiment involving the total of 12 Zeomic AJ10N samples. The first experiment results of the total of 12 samples blended with the type A zeolite (supporting 2.5 percent by weight of silver) by 0.5 percent by weight or 2.5 percent by weight are omitted because they were shown to be identical to the first experiment results of Zeomic AJ10N in Table 1 when measurement errors were considered.

It should be noted that the pH values for No. 1 to No. 12 in Table 1 were each obtained as an arithmetic mean based on a sample size of N = 3. The blending ratios in Table 1 each represent the blending ratio defined earlier. Also note that the values shown Table 2 and the tables that follow were each obtained as an arithmetic mean based on a sample size of N = 3, the same sample size applicable to the values shown in Table 1.

**[Table 1]**

| No | Silver zeolite (supporting 2.2 w% of silver) mixed in 100 g of water | | Citric acid mixed in 100 g of water | | Blending ratio | Mixture liquid of silver zeolite and citric acid Observed appearance and pH (/200 g) | | | | | |
|---|---|---|---|---|---|---|---|---|---|---|---|
| | Blending quantity pH | | Blending quantity pH | | | After 2 minutes | | After 10 minutes | | After 30 minutes | |
| | (g) | (-) | (g) | (-) | (-) | Appearance | pH | Appearance | pH | Appearance | pH |
| 1 | 1.0 | 9.6 | 0.80 | 2.4 | 0.8 | Cloudy | 4.4 | Containing deposits | 4.9 | Containing deposits | 4.9 |
| 2 | 1.0 | 9.6 | 0.90 | 2.4 | 0.9 | Cloudy | 44 | Translucent | 4.9 | Translucent | 4.9 |
| 3 | 1.0 | 9.6 | 1.00 | 2.4 | 1.0 | Cloudy | 4.4 | Translucent | 4.9 | Translucent | 4.9 |
| 4 | 1.0 | 9.6 | 1.20 | 2.4 | 1.2 | Clear | 4.3 | Clear | 4.9 | Clear | 4.9 |
| 5 | 1.0 | 9.6 | 1.30 | 2.4 | 1.3 | Clear | 42 | Clear | 4.9 | Clear | 4.9 |
| 6 | 1.0 | 9.6 | 1.50 | 2.4 | 1.5 | Clear | 4.2 | Clear | 4.5 | Clear | 4.5 |
| 7 | 5.0 | 9.8 | 4.00 | 2.3 | 0.8 | Cloudy | 4.4 | Containing deposits | 4.9 | Containing deposits | 4_{.}9 |
| 8 | 5.0 | 9.8 | 4.50 | 2.3 | 0.9 | Cloudy | 4.4 | Translucent | 4.9 | Translucent | 4.9 |
| 9 | 5.0 | 9.8 | 5.00 | 2.3 | 1.0 | Cloudy | 4.5 | Translucent | 4.8 | Translucent | 4.8 |
| 10 | 5.0 | 9.8 | 6.00 | 2.3 | 1.2 | Clear | 42 | Clear | 4.5 | Clear | 4.5 |
| 11 | 5.0 | 9.8 | 6.50 | 2.2 | 1.3 | Clear | 4.2 | Clear | 4.5 | Clear | 4.5 |
| 12 | 5.0 | 9.8 | 7.50 | 2.2 | 1.5 | Clear | 4.2 | Clear | 4.5 | Clear | 4.5 |

The results of the first experiment revealed that: when the blending ratio based on the blending quantity of citric acid to the blending quantity of silver zeolite was 0.8 or less, deposits of white unreacted silver zeolite form at the bottom of the translucent mixture liquid even after an elapse of 30 minutes after its mixing; when the blending ratio was 0.9 or greater but less than 1.2, deposits of unreacted silver zeolite did not form, but the mixture liquid was translucent even after an elapse of 10 minutes after its mixing; and when the blending ratio was 1.2 or greater but no greater than 1.5, the mixture liquid became clear 2 minutes after its mixing.

Based on the above, or specifically from the fact that deposits of unreacted silver zeolite would form when the blending ratio is 0.8 or less, it is clear that this amount of silver zeolite would be wasted. When the blending ratio is 0.9 or greater but less than 1.2, the mixture liquid would remain translucent even after an elapse of 30 minutes after its mixing; whereas, when the blending ratio is 1.2 or greater but no greater than 1.5, the mixture liquid would become clear 2 minutes after its mixing.

Based on these results, it is presumed that, in a mixture liquid based on a blending ratio of 0.9 or greater but less than 1.2, the citric acid almost entirely collapses, leaving a part of the crystal structure forming the ion exchange site of the silver zeolite, thereby causing nearly all silver ions to be eluted from the silver zeolite. In a mixture liquid based on a blending ratio of 1.2 or greater but no greater than 1.5, on the other hand, it is presumed that the crystal structure collapses completely and all silver ions are eluted from the silver zeolite. When effects on the skin, etc., are considered, it is undesirable that the blending ratio of the mixture liquid exceeds 1.5, because such liquid is strongly acidic as indicated by a pH of 4.4 or less and has adverse effects on the skin, and also because the citric acid is wasted.

Next, an experiment to examine the blending quantity of silver zeolite was conducted. To obtain a slurry of silver zeolite (dried at 110°C), up to 50 g of silver zeolite and up to 73 g of citric acid can be blended relative to 100 g of purified water. While the blending quantities explained above assume that the two are blended into 100 g of purified water as separate materials, an attempt was made to identify the maximum blending quantity of silver zeolite that would produce the silver ion antibacterial liquid proposed by the present invention. This maximum quantity of silver zeolite was 24 percent by weight, with citric acid blended by 28.8 percent by weight and purified water, by 47.2 percent by weight.

When an attempt was made to produce a mixture liquid at these blending quantities, silica gel generated at high concentration after the silver zeolite had reacted with the citric acid, and a mixture liquid could not be obtained. Accordingly, to determine the blending quantity of silver zeolite that would allow for commercial production of silver ion antibacterial liquid, the experiment described below was attempted by reducing the maximum blending quantity of 24 percent by weight. At room temperature (28°C), 100 g of purified water was put in a 200-g flask, after which citric acid was introduced by each of the six different blending quantities shown in Table 2 and dissolved completely. Next, a type A silver zeolite (supporting 2.5 percent by weight of silver and dried at 110°C) was introduced, under agitation, by each of the six different blending quantities shown in Table 2. The resulting mixture liquids were stored in 100-ml screw tubes by 100 g each, and the screw tubes were placed near a window and the properties of the mixture liquids were observed after 3 hours, 24 hours and 240 hours. The observed results are shown in Table 2.

**[Table 2]**

| | Blending ratio 1.2 | | Blending quantity of silver zeolite | After 3 hours | After 24 hours | After 240 hours |
|---|---|---|---|---|---|---|
| No | Silver zeolite (g) | Citric acid (g) | (Percent by weight) | Properties | Properties | Properties |
| 1 | 24.0 | 28.8 | 24 | Deposits of unreacted silver zeolite, clear | Gelatinous, blackish brown, opaque | Gelatinous, blackish brown, opaque |
| 2 | 20.0 | 24.0 | 20 | Liquid, clear | Gelatinous, blackish brown, opaque | Gelatinous, blackish brown, opaque |
| 3 | 15,0 | 18.0 | 15 | Liquid, clear | Gelatinous, blackish brown, opaque | Gelatinous, blackish brown, opaque |
| 4 | 10.0 | 12.0 | 10 | Liquid, clear | Liquid, clear | Gelatinous, blackish brown, opaque |
| 5 | 5.0 | 6.0 | 5 | Liquid, clear | Liquid, clear | Liquid, opaque |
| 6 | 2.5 | 3.0 | 2.5 | Liquid, clear | Liquid, clear | Liquid, clear |

**[Table 3]**

| | Blending ratio 0.9 | | Blending quantity of silver zeolite | After 3 hours | After 24 hours | After 240 hours |
|---|---|---|---|---|---|---|
| No | Silver zeolite (g) | Citric acid (g) | (Percent by weight) | Properties | Properties | Properties |
| 1 | 10.0 | 9.0 | 10 | Liquid, clear | Liquid, opaque | Gelatinous, brackish brown, opaque |
| 2 | 5.0 | 4.5 | 5 | Liquid, clear | Liquid, opaque | Gelatinous, brackish brown, opaque |
| 3 | 2.5 | 2.3 | 2.5 | Liquid, clear | Liquid, opaque | Liquid, translucent |

Sample No. 1 to No. 6 in Table 2, all associated with a blending ratio of 1.2 for the blending quantity of silver zeolite and that of citric acid, had different properties after 3 hours, 24 hours and 240 hours of mixing the two materials constituting each sample. With Sample No. 1, deposits of unreacted silver zeolite formed in a clear mixture liquid after 3 hours, and the liquid assumed a gelatinous, blackish brown, opaque appearance after 24 hours and 240 hours. The mixture liquids of Sample No. 2 and No. 3 changed from a clear liquid after 3 hours, to assume a gelatinous, blackish brown, opaque appearance after 24 hours and 240 hours. With Sample No. 4, the mixture liquid was clear after 3 hours and 24 hours, but looked like a blackish brown, opaque liquid after 240 hours. The mixture liquid of Sample No. 5 turned from a clear liquid after 3 hours and 24 hours, to an opaque liquid after 240 hours. With Sample No. 6, the mixture liquid remained clear even after 240 hours.

Sample No. 1 to No. 3 in Table 3, each having a blending ratio of 0.9 for the blending quantity of silver zeolite and that of citric acid, appeared similar to when the blending ratio of silver zeolite and citric acid was 1.2.

It was mentioned earlier that the "Method for Producing Silver Ion Antibacterial Liquid" as proposed by the invention under the prior application for patent had the problem of being troublesome because a process to remove the silica hydrate generated in the mixture liquid is required. However, the results shown in Table 2 and Table 3 indicate that, so long as the blending quantity of silver zeolite is 2.5 percent by weight or less, the mixture liquid would maintain a clear appearance after 240 hours, which brings to light that a silica hydrate removal process can be made unnecessary by simply adjusting the blending quantity of silver zeolite for producing the mixture liquid, to 2.5 or less percent by weight.

### (Second Experiment)

Next, the examples of the second experiment are explained.

In the second experiment, three types of silver zeolite supporting different quantities of silver (0.5, 2.5, and 5.0 percent by weight), respectively, but all manufactured according to the aforementioned method for manufacturing silver zeolite, as well as commercially available product Zeomic AJ10N (manufactured by Sinanen Zeomic Co., Ltd., supporting 2.2 percent by weight of silver and having an average particle size of approx. 2.5 µm), were used as samples. Then, for the aforementioned blending ratio based on the blending quantity of citric acid to that of silver zeolite, citric acid was weighed and taken to achieve a blending ratio of 0.6 for Sample No. 1 to No. 3, of 0.9 for Sample No. 4 to No. 6, of 1.1 for Sample No. 7 to No. 9, of 1.2 for Sample No. 10 to No. 12, and of 1.5 for Sample No. 13 to No. 15. Then, 100 g of a blended liquid prepared by blending each weighed silver zeolite into purified water, was mixed with 100 g of a blended liquid prepared by blending each weighed citric acid into purified water, to produce 200 g of a mixture liquid.

Then, 200 g of blended liquids, prepared by blending the silver zeolite and citric acid into purified water at the aforementioned blending quantities, respectively, were mixed under agitation to produce a mixture liquid. Since the chemical reaction involved normally reaches an equilibrium state in 24 hours, the silver ion concentration in the mixture liquid was measured 24 hours after its production. By the time 24 hours elapsed, deposits of unreacted silver zeolite had formed in the mixture liquid, so these deposits were filtered out and separated and the obtained liquid was measured for silver ion concentration using a high-frequency inductively-coupled plasma (ICP) emission spectrometer (ICP S-8100 manufactured by Shimadzu Corporation).

On the other hand, in Comparative Example No. 16 (No. 17 only in Table 6 (supported quantity of silver: 5.0 percent by weight)), 200 g of mixed liquid was prepared by mixing 100 g of a blended liquid constituted by purified water and silver zeolite blended in it, with 100 g of saline solution (sodium chloride 0.8 percent by weight), and the concentration of eluted silver ions was measured. The concentration was 450 to 590 ppb. It should be noted that, with this comparative example of No. 16, the silver ion concentration was measured by assuming the same conditions as those applicable to the spraying of silver zeolite (supporting 2.2 percent by weight of silver) for aerosol-type deodorizing cosmetic material on a sweaty body (containing 0.9 percent by weight of sodium chloride) as described in patent Literature 1.

Next, the blending ratios of silver zeolite and citric acid and the silver ion concentration are shown in Table 4 to Table 7 below for each test number representing one of four types of silver zeolite supporting different quantities of silver (supported quantity of silvers: 0.5, 2.5, 5.0 and 2.2 percent by weight).

**[Table 4]**

| No | Blending quantities of silver zeolite (supporting 0.5 w% of silver) and citric acid | | | |
|---|---|---|---|---|
| | Blending quantity of silver zeolite | Blending quantity of citric acid | Blending ratio | Silver ion concentration (Ag/100ml) |
| | (g) | (g) | (-) | (ppm) |
| 1 | 0.1 | 0.06 | 0.6 | 2.0 |
| 2 | 0.5 | 0.30 | 0.6 | 10.0 |
| 3 | 1.0 | 0.60 | 0.6 | 20.0 |
| 4 | 0.1 | 0.09 | 0.9 | 2.5 |
| 5 | 0.5 | 0.45 | 0.9 | 12.5 |
| 6 | 1.0 | 0.90 | 0.9 | 25.0 |
| 7 | 0.1 | 0.11 | 1.1 | 2.5 |
| 8 | 0.5 | 0.55 | 1.1 | 12.5 |
| 9 | 1.0 | 1.10 | 1.1 | 25.0 |
| 10 | 0.1 | 0.12 | 1.2 | 2.5 |
| 11 | 0.5 | 0.60 | 1.2 | 12.5 |
| 12 | 1.0 | 1.20 | 1.2 | 25.0 |
| 13 | 0.1 | 0.15 | 1.5 | 2.5 |
| 14 | 0.5 | 0.75 | 1.5 | 12.5 |
| 15 | 1.0 | 1.50 | 1.5 | 25.0 |
| 16 | 0.5 | 0.1 (salt water) | (-) | 0.5 |

**[Table 5]**

| No | Blending quantities of silver zeolite (supporting 2.5 w% of silver) and citric acid | | | |
|---|---|---|---|---|
| | Blending quantity of silver zeolite | Blending quantity of citric acid | Blending ratio | Silver ion concentration (Ag/100ml) |
| | (g) | (g) | (-) | (ppm) |
| 1 | 0.1 | 0.06 | 0.6 | 10.4 |
| 2 | 0.5 | 0.30 | 0.6 | 51.9 |
| 3 | 1.0 | 0.60 | 0.6 | 103.8 |
| 4 | 0.1 | 0.09 | 0.9 | 12.5 |
| 5 | 0.5 | 0.45 | 0.9 | 62.5 |
| 6 | 1.0 | 0.90 | 0.9 | 125.0 |
| 7 | 0.1 | 0.11 | 1.1 | 12.5 |
| 8 | 0.5 | 0.55 | 1.1 | 62.5 |
| 9 | 1.0 | 1.10 | 1.1 | 125.0 |
| 10 | 0.1 | 0.12 | 1.2 | 12.5 |
| 11 | 0.5 | 0.60 | 1.2 | 62.5 |
| 12 | 1.0 | 1.20 | 1.2 | 125.0 |
| 13 | 0.1 | 0.15 | 1.5 | 12.5 |
| 14 | 0.5 | 0.75 | 1.5 | 62.5 |
| 15 | 1.0 | 1.50 | 1.5 | 125.0 |
| 16 | 2.5 | 0.1 (salt water) | (-) | 0.6 |

**[Table 6]**

| No | Blending quantities of silver zeolite (supporting 5.0 w% of silver) and citric acid | | | |
|---|---|---|---|---|
| | Blending quantity of silver zeolite | Blending quantity of citric acid | Blending ratio | Silver ion concentration (Ag/100ml) |
| | (g) | (g) | (-) | (ppm) |
| 1 | 0.1 | 0,06 | 0.6 | 21.3 |
| 2 | 0.5 | 0.30 | 0.6 | 106.3 |
| 3 | 1.0 | 0.60 | 0.6 | 212.5 |
| 4 | 0.1 | 0.09 | 0.9 | 25.0 |
| 5 | 0.5 | (g) | 0.9 | 125.0 |
| 6 | 1.0 | 0.90 | 0.9 | 250.0 |
| 7 | 0.1 | 0.11 | 1.1 | 25.0 |
| 8 | 0.5 | 0.55 | 1.1 | 125.0 |
| 9 | 1.0 | 1.10 | 1.1 | 250.0 |
| 10 | 0.1 | 0.12 | 1.2 | 25.0 |
| 11 | 0.5 | 0.60 | 1.2 | 125.0 |
| 12 | 1.0 | 1.20 | 1.2 | 250.0 |
| 13 | 0.1 | 0.15 | 1.5 | 25.0 |
| 14 | 0.5 | 0.75 | 1.5 | 125.0 |
| 15 | 1.0 | 1.50 | 1.5 | 250.0 |
| 16 | 3.0 | 4.50 | 1.5 | 750.0 |
| 17 | 5.0 | 0.1 (salt water) | (-) | 0.6 |

**[Table 7]**

| | Blending quantities of Zeomic AJ01N (supporting 2.2 w% of silver) and citric acid | | | |
|---|---|---|---|---|
| No | Blending quantity of silver zeolite | Blending quantity of citric acid | Blending ratio | Silver ion concentration (Ag/100ml) |
| | (g) | (g) | (-) | (ppm) |
| 1 | 0.1 | 0.06 | 0.6 | 9.1 |
| 2 | 0.5 | 0.30 | 0.6 | 45.7 |
| 3 | 1.0 | 0.60 | 0.6 | 91.3 |
| 4 | 0.1 | 0.09 | 0.9 | 11.0 |
| 5 | 0.5 | 0.45 | 0.9 | 55.0 |
| 6 | 1.0 | 0.90 | 0.9 | 110.0 |
| 7 | 0.1 | 0.11 | 1.1 | 11.0 |
| 8 | 0.5 | (g) | 1.1 | 55.0 |
| 9 | 1.0 | 1.10 | 1.1 | 110.0 |
| 10 | 0.1 | 0.12 | 1.2 | 11.0 |
| 11 | 0.5 | 0.60 | 1.2 | 55.0 |
| 12 | 1.0 | 1.20 | 1.2 | 110.0 |
| 13 | 0.1 | 0.15 | 1.5 | 11.0 |
| 14 | 0.5 | 0.75 | 1.5 | 55.0 |
| 15 | 1.0 | 1.50 | 1.5 | 110.0 |
| 16 | 2.5 | 0.1 (salt water) | (-) | 0.6 |

Sample No. 4 in Table 4 indicates that, when the blending ratio was 0.9, the silver ion concentration was 2.5 ppm with respect to 0.1 g of silver zeolite blended. In addition, Sample No. 16 in Table 6 indicates that, when the blending ratio was 1.5, the silver ion concentration was 750.0 ppm with respect to 3.0 g of silver zeolite blended. Based on this, it is shown that the silver ion concentration would change in a range of 2.5 to 750 ppm at silver ion concentrations in a range of 0.01 to 3.0 percent by weight based on the blending quantity of silver zeolite.

No. 16 in Table 4, Table 5, and Table 7, and also No. 17 in Table 6, are comparative examples showing clearly lower silver ion concentration values of 0.5 to 0.6 ppm compared to other examples. On the other hand, the minimum value of silver ion concentration is 2.0 ppm for No. 1 to No. 15 in Table 4, Table 5, and Table 7, and No. 1 to No. 16 in Table 6, which are examples conforming to the present invention, and since this concentration is at least around four times the concentrations in the comparative examples, clearly these examples exhibit a higher bactericidal effect.

In Experiment Example 1 to Experiment Example 15 of the second experiment, the maximum blending quantity of weighed silver zeolite was 1.0 g, while the maximum blending quantity of weighed citric acid was 1.5 g, and in Table 7, No. 15 represents one such example where the supported quantity of silver was 5.0 percent by weight and the silver ion concentration was 250.0 pm. According to the application for use of the produced silver ion antibacterial liquid, its silver ion concentration can be adjusted in a desired manner based on the blending quantity of silver zeolite and the supported quantity of silver.

It should be noted that, as mentioned earlier, the commercial product TINOSAN SDC (brand name) is recognized as an excellent antibacterial agent as it contains a citric acid-silver complex and can be used safely as a paraben-free, alcohol-free antibacterial agent for skincare use. However, the TINOSAN SDC is too expensive for everyday use by general consumers and thus is not used as a popular product. For this reason, it is significant that a silver ion antibacterial liquid that contains a citric acid-silver complex, and which does not turn blackish brown when irradiated with light, can be produced from a silver zeolite without using a silica hydrate removal process.

The products in the mixture liquids are explained further below.

As mentioned earlier, the structural formula of silver zeolite is as follows:

(αNa₂ βAg₂)O·Al₂O₃-2SiO₂nH₂O (α + β = 1, n = 5; dried at 110°C)

The crystal structure forming the ion exchange site of the silver zeolite is such that the silver ion is electrostatically bonded to the Al part in the three-dimensionally bonded Si-O-Al-O-Si crystal structure, and reportedly the silver ion in the above crystal structure elutes as a result of ion exchange action to kill bacteria. In other words, the structure of type A silver zeolite is that of aluminosilicate comprising silica (SiO₂) and alumina (Al₂O₃), whose framework is characterized by a crystal structure based on three-dimensionally bonded (AlO₄)⁻ tetrahedral and (SiO₄)⁻ tetrahedral, with the silver ion electrostatically adsorbed to the Al part.

The process of how type A silver zeolite is collapsed by citric acid is considered as follows:
1. The proton of citric acid undergoes ion exchange with the sodium ion present at the negatively charged position on the (AlO₄)⁻ tetrahedral of type A silver zeolite (because the selectivity coefficient of the sodium ion is smaller than that of the silver ion).
2. The excess proton acts upon and severs the Al-O bond in the framework.
3. The severance of the Al-O bond causes the framework structure to collapse, and the silver ion, sodium ion, etc., that have been adsorbed onto the silver zeolite are released into the solution.
4. The released silver ion reacts with the citric acid to produce a citric acid-silver complex.
5. The aluminum reacts with C₆H₅O₇³⁻ of citric acid to produce a citric acid-aluminum complex.
6. The citric acid-silver complex partially dissociates in water, causing a very small quantity of silver ions to also be present in water.
7. Sodium is present as ions in water.
8. Silicon is suspended or deposited as silica gel. The suspended/deposited silica gel has a small quantity of silver ions adsorbed onto its surface.

In consideration of the aforementioned chemical reaction, the products contained in the mixture liquid likely include citric acid-silver complex, silica hydrate, citric acid-aluminum complex, and silver ions. However, although presence of citric acid-silver complex in the silver ion antibacterial liquid cannot be identified, it is clear from the ionization of all silver ions in the silver zeolite that a bactericidal system function, different from what has been heretofore known, is at work.

As for the method for producing silver ion antibacterial liquid as proposed by the present invention, it was revealed that, by weighing silver zeolite and taking a blending quantity of it corresponding to 0.01 to 3.0 percent by weight, and then weighing citric acid and taking a blending quantity of it so that the aforementioned blending ratio falls in a range of 0.9 to 1.5, followed by blending the two materials into purified water and mixing them, a silica hydrate removal process is no longer necessary because the mixture liquid would not turn blackish brown but remain translucent or clear in its appearance after 240 hours as shown in Table 2 and Table 3. Going forward, when silver ion antibacterial liquid containing a citric acid-silver complex is to be prepared, such silver ion antibacterial liquid can be produced trouble-free without a need for silica hydrate removal process by weighing silver zeolite and taking a blending quantity of it corresponding to 0.01 to 3.0 percent by weight and then weighing citric acid and taking a blending quantity of it so that the blending ratio of the citric acid to the silver zeolite by weight becomes 0.9 to 1.5, and then blending the two materials into purified water and mixing them.

The blending quantities (in percent by weight) of silver zeolite, citric acid, and purified water mentioned above are such that the silver zeolite is blended by 0.01 to 3.0 percent by weight, the citric acid is blended by a given percent by weight so that its blending ratio to the silver zeolite falls in a range of 0.9 to 1.5, and the purified water accounts for the remaining percent by weight to give a total of 100 percent by weight, and the silver zeolite and citric acid are blended into the purified water and mixed accordingly. Why the silver ion antibacterial liquid thus produced does not turn blackish brown after 240 hours of irradiation with light is considered.

As mentioned earlier, the silica hydrate is removed from the silver ion antibacterial liquid because silver hydroxide (AgOH) is adsorbed to the surface of the silica hydrate and silica hydrate silver hydroxide generates, in which case grains of the produced silica hydrate silver hydroxide agglutinate together and the agglutinated product becomes silver oxide (Ag₂O; blackish brown color) when irradiated with light; hence the need to remove the silica hydrate. However, while the silver ion antibacterial liquid produced at the blending quantities mentioned above does produce silver oxide due to light because of generation of the aforementioned silica hydrate silver hydroxide, the silver ion antibacterial liquid contains only a trace amount of this silica hydrate silver hydroxide and therefore grains of the silica hydrate silver hydroxide do not agglutinate together, which means that, although this unagglutinated silica hydrate silver hydroxide may become silver oxide due to light, the silver adsorption density at the surface of the silica hydrate silver hydroxide is small, and this, coupled with the size of silver oxide which is too small at 0.1 mm or less to be visible to the naked eye, its blackish brown color is presumed to be visually recognized by humans, as shown by the properties after 240 hours in Table 2 and Table 3. It is said that the size visible to the naked eye is 0.1 mm (refer to http://microscopelabo.jp/learn/025/).

It should be noted that, because the silver ion antibacterial liquid as proposed by the present invention can be powderized into a silver ion antibacterial powder, which allows this silver ion antibacterial powder to be reduced with purified water for use as silver ion antibacterial liquid later on, the method for producing silver ion antibacterial powder is explained below.

### (Method for Producing Silver Ion Antibacterial Powder)

To produce a silver ion antibacterial powder as a powder form of silver ion antibacterial liquid, any method can be adopted whereby the silver ion antibacterial liquid is freeze-dried using a decompression freeze-drying machine, or spray-dried using a decompression spray-drying machine. For example, when silver ion antibacterial liquid is produced from 11.0 g of silver zeolite (supporting 2.5 percent by weight of silver and dried at 110°C) and 13.2 g of citric acid, this silver ion antibacterial liquid can be freeze-dried using a decompression freeze-drying machine to obtain 24.2 g of silver ion antibacterial powder.

When 1.0 g of the silver ion antibacterial powder thus obtained was dissolved in 1,000 g of water, it dissolved completely and the resulting silver ion concentration was 11.5 ppm.

Next, examples of the method for producing silver ion antibacterial liquid are explained.

### (Example 1 of First Process)

A commercially available silver zeolite (Zeomic AJ10N manufactured by Sinanen Zeomic Co., Ltd., having an average particle size of 2.5 µm, supporting 2.2 percent by weight of silver, and dried at 100°C) was weighed and 0.150 kg of it was taken, and citric acid powder was weighed and 0.135 kg of it was taken. Then, 149.71 kg of purified water was introduced to a stainless steel tank with a capacity of 500 liters and agitated. Thereafter, the citric acid powder was introduced and dissolved completely. After that, the silver zeolite was introduced. The mixture liquid thus obtained had a silver zeolite concentration of 0.1 percent by weight, and its blending ratio of silver zeolite/citric acid was 0.9. The mixture liquid became clear after 5 minutes of mixing and agitation, but this mixture liquid was agitated further by 30 minutes. This way, the silver ion antibacterial liquid proposed by the present invention was manufactured. This silver ion antibacterial liquid had a silver ion concentration of 22 ppm. By spray-coating this silver ion antibacterial liquid onto a cotton non-woven fabric to impregnate the fabric with the liquid, an antibacterial wet sheet can be manufactured.

### (Example 2 of First Process)

The silver zeolite manufactured in Manufacturing Example 2 above (supporting 2.5 percent by weight of silver and dried at 110°C) was weighed and 6.49 kg of it was taken, and citric acid powder was weighed and 9.73 kg of it was taken. Then, 199.78 kg of purified water was introduced to a stainless steel tank with a capacity of 500 liters and agitated. Thereafter, the citric acid powder was introduced and dissolved completely. After that, the silver zeolite was introduced. The mixture liquid thus obtained had a silver zeolite concentration of 3.0 percent by weight, and its blending ratio of silver zeolite/citric acid was 1.5. The mixture liquid became clear after 5 minutes of mixing and agitation, but this mixture liquid was agitated further by 30 minutes. This way, the silver ion antibacterial liquid proposed by the present invention was manufactured. This silver ion antibacterial liquid had a silver ion concentration of 750 ppm. By blending this silver ion antibacterial liquid into a makeup preparation as an antiseptic liquid, a makeup preparation with added antibacterial property can be manufactured.

### (Example of Second Process)

The silver zeolite manufactured in Manufacturing Example 3 above (supporting 5.0 percent by weight of silver and dried at 110°C) was weighed and 0.875 kg of it was taken, and citric acid powder was weighed and 1.313 kg of it was taken. Next, 50 kg of purified water was introduced to a stainless steel tank with a capacity of 500 liters and agitated. Thereafter, the 1.313 kg of citric acid powder was introduced and dissolved completely. After that, the 0.875 kg of silver zeolite was introduced. The mixture liquid thus obtained had 1.75 percent by weight of silver zeolite blended in it, the silver ion concentration was 875 ppm, and its blending ratio of silver zeolite/citric acid was 1.5. The mixture liquid became clear after 5 minutes, but this mixture liquid was agitated further by 30 minutes. Thereafter, 100 kg of purified water was introduced to dilute the liquid, which was then agitated for 18 hours. This way, the silver ion antibacterial liquid proposed by the present invention was manufactured. This silver ion antibacterial liquid had a silver ion concentration of 250 ppm.

It should be noted that, if the silver ion concentration of the mixture liquid exceeds 750 ppm in the example of the second process, purified water must be introduced by no later than 3 hours after the preparation of the mixture liquid. Since the silica hydrate will agglutinate after 3 hours, it is safe to introduce purified water within 2 hours if possible.

It should be noted that, although the blending quantities were explained in percent by weight, the unit need not be limited to this and clearly the percent by weight values can be converted to percent by volume values based on the density, and therefore the method for converting to percent by volume values is not explained.

For reference, the density of silver zeolite is 1.99 g/cm² and that of citric acid is 1.665 g/cm².

The silver ion antibacterial liquid proposed by the present invention is used for the purpose of preventing or defending against bacteria, viruses, and other microorganisms spreading and transmitting diseases, etc., to the human body, where it is used directly for antibacterial cleaning, mixed into the materials of antibacterial products to add an antibacterial effect to such products, or blended into or sprayed or dip-coated onto antibacterial products to add an antibacterial effect to such products. These products with added antibacterial effect are hereinafter referred to as "silver ion-containing products." Specific examples of use of these silver ion-containing products include liquid cosmetic preparations, wet tissues, liquid deodorants, liquid bathing agents, liquid laundry softeners, etc.

In addition, the silver ion antibacterial agent proposed by the present invention may be coated or deposited onto clothes, linens, and tools used or worn by medical professionals, nurses, patients, etc., in hospitals for the purpose of preventing or defending against in-hospital infections, or onto wet tissues, wet sheets, masks, and other sundries used in everyday life, or installations, equipment, and tools of public facilities, etc., construction materials, fittings, and fixtures, ceilings, walls, baseboards, floors, handrails, handrails of staircases, toilet seats, bathtubs, washing basins, washing tools, etc., of general building structures, for the purpose of preventing or defending against infections by influenza and other viruses. These articles with added antibacterial effect used in hospitals and public places are hereinafter referred to as "silver ion-containing treated products."

### (Silver Ion-containing Products)

For the base material of a liquid-absorbent sheet that can retain water and is flexible and used for an antibacterial wet sheet being a silver ion-containing product according to the present invention, a natural fiber such as pulp, synthetic fiber such as rayon, polypropylene, polyvinyl alcohol, polyester, or polyacrylonitrile, synthetic pulp made of polyethylene, etc., or inorganic fiber such as glass wool, or the like is used.

The silver ion antibacterial liquid mentioned above is impregnated into or sprayed onto the base material of liquid-absorbent sheet to prepare an antibacterial wet sheet. This antibacterial wet sheet can be used for cleaning purposes such as wiping the bottoms of young children, elderly adults, etc., when changing diapers or adult diapers or cleaning the skin as wet towels, or for pet care purposes as easy cleaning solutions that can simply wipe away hairs, soiling and body odor of pets, or the silver ion antibacterial liquid can also be blended into products selected from the group that includes liquid cosmetic preparations, wet tissues, liquid deodorants, liquid bathing agents and liquid laundry softeners to make silver ion-containing products.

### (Silver Ion-containing treated Products)

The silver ion antibacterial liquid mentioned above can also be coated or deposited onto the exterior casings of various medical equipment, or door knobs, bedside tables, bed frames and various handrails, etc., frequently contacted by hands, to make silver ion-containing treated products for preventing infections in medical facilities or public places.

Incidentally, regarding the silver ion concentration, "Ginkagoubutsu no Koukinkouka Kenshou ni Kansuru Kisokenkyuu (Basic Research on Verification of Antibacterial Effect of Silver Compounds)" (refer to Boukin Boukabi, Vol. 37, No. 7, p. 499) by Sakagami et al. reports that the bacterial count of the Staphylococcus aureus decreased by 2 logs or more at a silver ion concentration of 10 ppb in a condition where nutrient broth had been added (this is referred to as "NB culture," where animal peptone and beef extract are used as the base materials of the culture medium). This means that, so long as the silver ion concentration is 10 ppb (corresponding to 0.01 ppm), the silver ions would demonstrate an antibacterial effect and Staphylococcus aureus would die.

The result of each test conducted on the functionality of the silver ion antibacterial liquid proposed by the present invention is shown below.

### (Test of Antibacterial Power Efficacy)

Two hundred milliliters of antibacterial liquid was prepared from 5 g of Zeomic AJ10N (manufactured by Sinanen Zeomic Co., Ltd., supporting 2.2 percent by weight of silver and having an average particle size of approx. 2.5 µm) and 6 g of citric acid. Since this antibacterial liquid had a blending ratio of 1.2 and was corresponding to Sample No. 10 in Table 1, it was presumed that all silver ions eluted from the silver zeolite. When the antibacterial test was conducted by using this antibacterial liquid as the test solution (silver ion concentration: 550 ppm) as well as liquids in which the Staphylococcus aureus, Pseudomonas aeruginosa, Escherichia coli, and Candida albicans were suspended, respectively (bacterial concentration: 10⁶ to 10⁷ CFU/ml), the counts of live remaining bacteria were 10 or less 30 minutes after the inoculation.

Thereafter, 1.85 ml of the antibacterial liquid was dissolved in 100 ml of purified water for use as the test solution (silver ion concentration: 10 ppm). It should be noted that a phosphoric acid buffer solution was needed to prevent sudden changes in pH and osmotic pressure in microbial cells and maintain microorganisms in an active state.

In this test, 10 ml of the test solution was taken into a test tube. At this point, 0.1 ml of each of the Staphylococcus aureus-, Pseudomonas aeruginosa-, Escherichia coli-, and Candida albicans-suspended liquids (bacterial concentration: 10⁶ to 10⁷ CFU/ml) was inoculated to the test solution in the test tube. After that, 1.0 ml of specimen was sampled immediately, 1 minute, 3 minutes, 10 minutes and 30 minutes after the inoculation, and each specimen was diluted with 9 ml of phosphoric acid buffer solution. Then, 1.0 ml of the diluted specimen liquid was taken and diluted with 9 ml of phosphoric acid buffer solution. This diluting operation was repeated four times and 1.0 ml of each serially diluted specimen was inoculated to a standard agar culture. After two days of culturing at 36°C, the number of colonies formed on the surface was counted to measure the count of live remaining bacteria. The results are shown in Table 8.

**[Table 8]**

| Bacterial strain | S. aureus | P. aerug | E. coli | C. albic |
|---|---|---|---|---|
| Immediately after | 1.5×10⁵ | 1.9×10⁵ | 2.2×10⁵ | 2.8×10⁻⁵ |
| After 1 minute | 3.4×10⁻⁴ | 1.8×10⁴ | 6.8×10³ | 1.2×10⁵ |
| After 3 minutes | 2.8×1.0³ | 6.5×10² | 4.8×10¹ | 1.3×10⁴ |
| After 10 minutes | 10 or less | 10 or less | 10 or less | 6.8×10² |
| After 30 minutes | 10 or less | 10 or less | 10 or less | 10 or less |

### (Test Results)

In general, tests that use an inorganic silver antibacterial agent (silver zeolite) utilize the elution of silver ions due to ion exchange with cations present in water, which means that gradually eluting silver ions kill bacteria and therefore antibacterial property is recognized after six hours or so at the earliest. In this test, however, bacteria died in units of minutes and bacterial death was evident after 10 minutes. Based on this, it is presumed that a bactericidal system function, different from what has been heretofore known, is at work as a result of elution of all silver ions from the silver zeolite. And, it was revealed that, at a silver ion concentration of 10 ppm, the present invention would achieve excellent immediate antibacterial power against Gram-positive bacteria, Gram-negative bacteria, and yeasts.

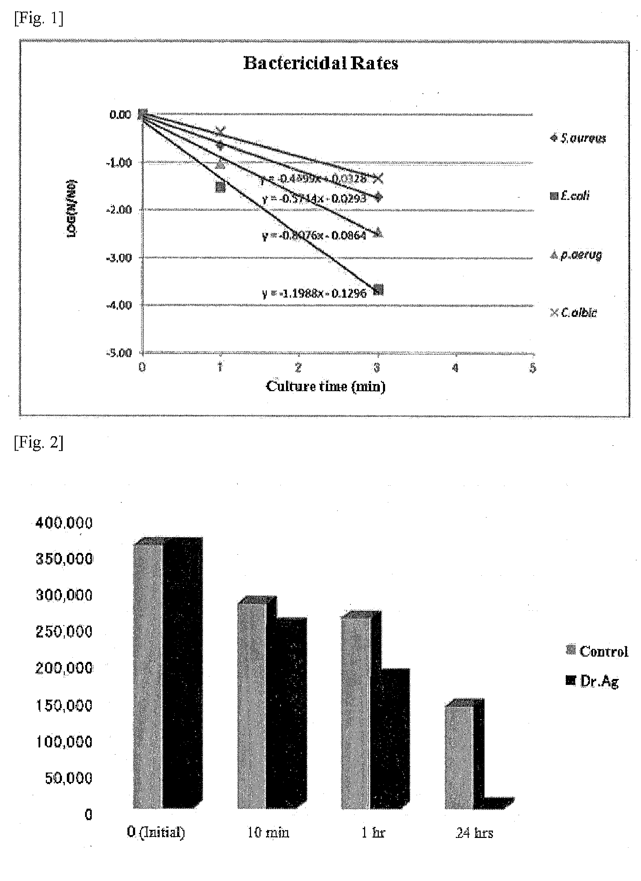

For reference, the bactericidal rate against each bacterium achieved by the silver ion antibacterial liquid proposed by the present invention (silver ion concentration: 10 ppm) is shown in Fig. 1.

In the above, the bacteria used in the antibacterial test are roughly representative of various microorganisms. For example, the Escherichia coli is a Gram-negative bacillus belonging to the facultative anaerobe group present in the environment. This bacterium is often used as an indicator bacterium of contamination. One characteristic feature of the cellular membrane structures of Gram-negative bacteria is that it has lipopolysacchaudes covering a thin peptide glycan layer and outer membrane on the outer side of the peptide glycan layer. Many of these bacteria are harmless, but some have strong toxicity such as the O-157. Silver ions easily penetrate through this thin peptide glycan layer to demonstrate a bactericidal effect.

The Staphylococcus aureus is a representative Gram-positive coccus belonging to the facultative anaerobe, having a thick peptide glycan layer on its surface and a cellular membrane on the inner side of this layer. Accordingly, silver ions do not demonstrate an antibacterial effect as easily as they do against Gram-negative bacteria. This bacterium is present in humans and animals in the skin and among the resident bacteria in the digestive tract (intestine) (bacterial flora in the intestine). This bacterium also triggers infectious diseases in humans, including various skin infections such as abscess, food poisoning, as well as fatal infections, diseases such as pneumonia, meningitis, and blood poisoning. Staphylococcus aureus bacteria resistant to antibiotics such as Methicillin are called "MRSA" that cause in-hospital infections.

The Pseudomonas aeruginosa is a Gram-negative bacillus which causes in-hospital infections just like the MRSA. The Pseudomonas aeruginosa may cause infectious diseases in patients whose immune system has weakened. The most frightening aspect of this bacterium is that it has "multidrug resistance" and resists many antibiotics.

Unlike the aforementioned bacteria, the Candida albicans is a yeast. Its cellular wall is formed by polymerized polysaccharides including glucan, chitosan, mannan, and chitin. It is well known that the Candida albicans invades the submucosal tissues and causes hematogenous and lymphogenous infections of the organs.

What the bactericidal rate tells us is the number of digits by which the count of live remaining bacteria decreases per minute. For example, the bactericidal rate of the Escherichia coli is approx. 1.2 based on this graph, which means that theoretically 1.2 x 5 = 6.0 digits, or specifically 99.9999%, of the initial bacteria will die. This indicates that, while normal silver-based inorganic antibacterial agents demonstrate an antibacterial effect in units of hours, the present invention achieves a dramatically higher bactericidal speed.

Next, the measured results of how long the silver ion antibacterial liquid proposed by the present invention took to kill bacteria are shown.

The measuring conditions are specified below.
Sample: Silver ion antibacterial liquid (silver ion concentration: 20 ppm)
Bacterial strain used: Staphylococcus aureus (concentration of bacteria-suspended liquid: 10⁷ cfu/ml)
Test method: 5.0 ml of silver ion Antibacterial liquid and 5.0 ml of nutrient liquid (concentration: 1/20, NB culture) were mixed, and then 10 ml of Staphylococcus aureus-suspended liquid was inoculated to the mixture, after which the inoculated liquid was kept still and cultured at room temperature, and the live bacterial count was measured over time. (Silver ion concentration in the test solution: 10 ppm)

In the meantime, a control was produced by mixing 5.0 ml of silver ion antibacterial liquid and 5.0 ml of sterile water, and then 10 ml of Staphylococcus aureus-suspended liquid was inoculated to the mixture, and the live bacterial count was measured over time.

For reference, the culture solution was collected initially (0 minute) and after 1 minute, 3 minutes, 10 minutes, and 30 minutes, and a sample was taken from each collected sample to measure the bacterial count.

The measured results of the above are shown in Table 9. These measured results show the relationship of the nutrient concentration and bacterial deaths of the Staphylococcus aureus. For reference, the measured values are average bacterial counts based on a sample size of N = 3, expressed in the unit of bacterial concentration (cfu/ml).

**[Table 9]**

| Nutrient concentration | Initial | After 1 minute | After 3 minutes | After 10 minutes | After 30 minutes |
|---|---|---|---|---|---|
| 1/20 NB | 1.5×10⁵ | 3.4×10⁴ | 2.8×10³ | 2.0×10 | 10 or less |
| Sterile water | 1.6×10⁵ | 1.3×10⁴ | 5.7×10² | 10 or less | 10 or less |

Based on these results, the bacterial count dropped to or below the detection limit after 30 minutes, even in a nutrient-rich environment, showing a high antibacterial capability of the silver ion antibacterial liquid diluted two-fold (silver ion concentration: 10 ppm).

The fact that the concentrations of nutritional constituents are far lower in a general living environment confirms practical utility of the silver ion antibacterial liquid proposed by the present invention.

Next, attention is drawn to the problem of elderly adults, young children, and other patients whose body resistance has weakened, becoming infected in hospitals by so-called multidrug-resistant bacteria having resistance to multiple drugs. Bacteria causing these infections include the Staphylococcus aureus (MRSA), Pseudomonas aeruginosa, and Tubercle bacillus, for example. While healthy persons can sufficiently resist these bacteria through the neutrophils in the blood stream, they can cause blood poisoning and other tragic situations once in the blood stream of patients whose immune system and physical strength have weakened.

One serious example of in-hospital infection in recent years relates to the problem of the multidrug-resistant Tubercle bacillus. Accordingly, in trying to cut off the path of infection of the multidrug-resistant Tubercle bacillus, it was considered that killing the bacteria attached to pillow cases, sheets, etc., using the aforementioned silver ion antibacterial liquid could prevent the damage from spreading.

Therefore, a test was conducted to examine whether the silver ion antibacterial liquid had a bactericidal effect against the Tubercle bacillus. The measuring conditions are specified below.

Sample: Silver ion antibacterial liquid (silver ion concentration: 20 ppm) Bacterial strain used: Mycobacterium bovis (BCG) RIMD1314006 (Tubercle bacillus bovine: mutant strain of BCG, concentration of bacteria-suspended liquid: 10⁷ cfu/ml)

Test method: 0.1 ml of Tubercle bacillus-suspended liquid was inoculated to 10 ml of silver ion antibacterial liquid and the inoculated liquid was kept still and cultured at room temperature, with the live bacterial count measured over time. For reference, the bacterial count was measured using the MF method by collecting 1 ml of culture solution and adding it to 9 ml of SCDLP bouillon culture to stop the bactericidal action.

The culture solution was collected initially (0 minute) and after 10 minutes, 1 hour and 24 hours.

The measured results of the above are shown in Table 10. For reference, the unit of measured values is the bacterial concentration (cfu/ml) and the detection limit is 10 cfu/ml.

**[Table 10]**

| | Initial | After 10 minutes | After 1 hour | After 24 hours |
|---|---|---|---|---|
| Sterile saline solution (Control) | 3.6 10⁵ | 2.8×10⁵ | 2.6×10⁵ | 1.4×10⁵ |
| Silver ion antibacterial liquid proposed by the present invention (Trademarked "Dr. Ag") | 3.6×10⁵ | 2.5×10⁵ | 1.8×10⁵ | 4.7×10³ |
| Rate of decrease (%) | 0 | 11 | 31 | 97 |

It should be noted that the results of Table 10 are shown in Fig. 2 for clarity. Additionally, the killing curve of the Tubercle bacillus is shown in Fig. 3.

These results show that the silver ion antibacterial liquid has antibacterial capability against the Tubercle bacillus.

Influenza causes a social problem by creating an epidemic every winter. Recently influenza viruses have emerged that are resistant to drugs. These influenza viruses undergo H275Y resistant mutation in the NA protein and exhibit drug resistance to Oseltamivir (Tamiflu) and Peramivir (Rapiacta).

Infection with influenza viruses occur as they are adsorbed to the sialic acid in the end molecules of glycoproteins at the surface of host cells. The virus spikes or surface proteins called the hemagglutinin (HA) and neuraminidase (NA) play important roles in this process. These proteins are called spikes because they look like spikes stuck in the envelope (shell) of the virus. The type A influenza viruses have different types of spikes depending on the combination of constituent amino acids--there are 16 types of HA and nine types of NA--and these combinations make the type A influenza viruses very diverse, creating 144 "subtypes" from H1N1 to H16N9.

However, silver ions are not affected by these different types of spikes in that they adsorb to and alter all spike proteins, thus preventing influenza viruses from adsorbing to the surface of host cells. In other words, silver ions can completely defend against infection of influenza viruses.

Accordingly, a test was conducted to examine whether the silver ion antibacterial liquid proposed by the present invention (silver ion concentration: 20 ppm) had an inactivation effect against a type A influenza virus (H1N1). This test was conducted at Kitasato Research Center for Environmental Science.

### (Virus Inactivation Test)

After inoculating 0.1 ml of type A influenza virus (H1N1) to 0.9 ml of the silver ion antibacterial liquid proposed by the present invention (silver ion concentration: 20 ppm) and agitating the mixture gently using a test tube mixer, the antibacterial liquid was caused to act upon the virus for 1 minute or 5 minutes at room temperature. After a specified time, 0.1 ml of the mixture was collected and diluted 100-fold using a Dulbeccos Modified Eagles Medium (DMEM) containing 0.2% bovine fetal serum (FBS) for use as the undiluted liquid for measuring virus liquid infection. For reference, the initial virus infectivity titer was measured immediately after the virus liquid was inoculated to a citric acid solution (pH 6.1) used as the control, in order to examine the effect of pH, as well.

### (Method for Measuring Virus Infectivity Titer)

The undiluted liquid for measuring virus infectivity titer was diluted in 10 stages using phosphoric acid buffer saline solution (PBS), after which 50 µL of the undiluted liquid for measuring infectivity titer and dilute virus liquid thereof, and 50 µL of canine kidney-derived cells (MDCK) suspended in 5% FBS-spiked DMEM, were seeded in 96-hole microplates. Thereafter, the samples were cultured for four days in a carbonic acid gas incubator controlled at 37°C. The cultured samples were observed under an inverted microscope for cytopathic effect (CPE), and the virus infectivity titer (TCID₅₀/ml) was obtained using the Reed-Muench method.

The results are shown in Table 11.

**[Table 11]**

| Sample | Acting time Decrease in logarithm of infectivity titer | | | | |
|---|---|---|---|---|---|
| | Initial | After 1 minute | After 5 minutes | After 1 minute | After 5 minutes |
| Aqueous solution of citric acid of pH 6.1 (Control) | 7.2 × 10⁵ | 1.0 × 10⁶ | 5.4 × 10⁵ | 0 | 0 |
| Silver ion antibacterial liquid adjusted to pH 6.1 | | 6.3 × 10¹ or less | 6.3 × 10¹ or less | 4.1 or less | 4.1 or less |

According to these test results, causing the silver ion antibacterial liquid (silver ion concentration: 20 ppm) to act upon the influenza virus led to a drop in the infectivity titer to or below the detection limit (6.3 x 10¹TCID₅₀/ml) after 1 minute. The logarithm of virus infectivity titer decreased by 4.1 log₁₀ or more from the initial infectivity titer (rate of decrease: 99.99% or more). The foregoing confirms a performance of the silver ion antibacterial liquid to defend against the influenza virus.

Normally for materials, a decrease in the logarithm of the test bacteria by 2.0 or more is used as the judgment criterion to determine that they have an antibacterial effect. On the other hand, the same judgment criterion is 4.0 or more for antiseptic solutions. Assessing from this, the silver ion antibacterial liquid (silver ion concentration: 20 ppm) may rather be categorized as an antiseptic solution.

## Claims

1. A method for producing a silver ion antibacterial liquid where the silver ion antibacterial liquid to be produced contains silver ions eluted from a silver zeolite supporting 0.5 to 5.0 percent by weight of silver, wherein said method for producing silver ion antibacterial liquid is **characterized in that** it involves:
using the silver zeolite, citric acid, and purified water and implementing a first process comprising weighing the silver zeolite, citric acid, and purified water, and taking a blending quantity of each, and then agitating and mixing a blended liquid prepared by blending the silver zeolite, citric acid, and purified water to prepare a mixture liquid, or a second process comprising weighing and taking a blending quantity of each of the above, preparing the mixture liquid mentioned above, and then adding purified water to dilute the mixture liquid and prepare a diluted liquid;
wherein, the blending ratio of the citric acid to the silver zeolite corresponds to a blending quantity in a range of 0.9 to 1.5 when the mixture liquid is prepared in the first and second process, and the silver ion concentration of the produced silver ion
antibacterial liquid is 2.5 to 750 ppm.

2. A method for producing a silver ion antibacterial liquid according to Claim 1, **characterized in that** the blending quantity of the silver zeolite is 0.01 to 3.0 percent by weight in the first process, while the blending quantity of the silver zeolite exceeds 3.0 percent by weight in the second process.

3. A method for producing a silver ion antibacterial liquid according to Claim 1, **characterized in that** the silver zeolite is a type A or type X silver zeolite.

4. A silver ion antibacterial liquid produced by a method for producing a silver ion antibacterial liquid according to Claim 1.

5. A silver ion antibacterial powder **characterized in that** a silver ion antibacterial liquid according to Claim 1 is powderized by freeze-drying or spray-drying.

6. A silver ion-containing product **characterized in that** a silver ion antibacterial liquid according to Claim 4 is blended into a product selected from the group that includes liquid makeup preparations, wet tissues, liquid deodorants, liquid bathing agents, and liquid laundry softeners.

7. A silver ion-containing treated product **characterized in that** a silver ion antibacterial liquid according to Claim 4 is coated or deposited onto articles used in hospitals and public places.
